# DEMANDE DE BREVET EUROPEEN

(11) **EP 4 410 355 A1**
(43) Date de publication de la demande: **07.08.2024**
(21) Numéro de dépôt: 24154929.4
(22) Date de dépôt: 31.01.2024
(51) Int. Cl.: A61M 39/10, A61M 39/16, A61M 39/18, F16L 37/084

(54) **DISPOSITIF DE RACCORDEMENT ASEPTIQUE D'UN TUBE**

(30) Priorité: 02.02.2023 FR 2301005
(71) Demandeur: Parker Hannifin EMEA S.à.r.l., 1163 Etoy (CH)
(72) Inventeur: BINDER, Florent, 35000 RENNES (FR)
(74) Mandataire: Cabinet Boettcher

(57) **Abrégé**

Dispositif (10) de raccordement aseptique d'un tube (100, 200), comprenant :
- un corps (20, 30, 40) comportant des moyens de couplage (20, 40) à un autre dispositif (10) de raccordement et un embout (30) tubulaire qui est agencé pour être engagé dans le tube, un canal (31) s'étendant longitudinalement dans l'embout et débouchant dans un logement formé dans le corps et ouvert sur une surface dudit corps opposée à l'embout, le logement recevant un élément d'étanchéité (50) ;
- un tiroir (60) de protection de l'élément d'étanchéité, monté coulissant dans le corps parallèlement à ladite surface entre une position d'obturation du logement et une position de dégagement du logement ; et
- des moyens d'encliquetage (24, 63) à l'autre dispositif de raccordement, agencés pour empêcher, lors du couplage du dispositif de raccordement avec l'autre dispositif de raccordement, un désaccouplement dudit dispositif de raccordement et dudit autre dispositif de raccordement.

## Description

La présente invention concerne le domaine du transport de fluides liquides ou gazeux et plus particulièrement un dispositif de raccordement aseptique destiné à réaliser le raccordement de deux tubes stériles.

### ARRIERE PLAN DE L'INVENTION

On connaît des ensembles de raccordement destinés à connecter des canalisations stériles reliées à des éléments de circuits de l'industrie pharmaceutique, tels que des filtres, des centrifugeuses, des pompes, des réservoirs en plastique souple ou des conteneurs de traitement comme des bio-processeurs.

Un tel ensemble comprend généralement deux dispositifs de raccordement qui sont destinés à être connectés entre eux après que chacun ait été raccordé à une extrémité de canalisation. Chaque dispositif de raccordement comprend :
- un corps pourvu d'un embout tubulaire agencé pour être engagé dans l'extrémité d'une canalisation, un canal s'étendant longitudinalement dans l'embout et débouchant dans un logement formé dans le corps et ouvert sur une surface du corps, et le logement recevant un élément d'étanchéité destiné à rentrer en contact étanche avec l'élément d'étanchéité de l'autre dispositif de raccordement ;
- un couvercle de protection de l'élément d'étanchéité, monté coulissant sur le corps parallèlement à la surface du corps entre une position d'obturation du logement et une position de dégagement du logement ; et
- des moyens de couplage à l'autre dispositif de raccordement.

Lors de la connexion de tels dispositifs entre eux, les couvercles de protection sont amenés à passer de la position d'obturation à la position de dégagement sous l'effet d'un effort transversal exercé par les corps des dispositifs afin de réaliser une mise en contact étanche des éléments d'étanchéité entre eux.

Si les dispositifs de raccordement comprennent des moyens de verrouillage de leur corps en position connectée, rien n'empêche l'opérateur d'interrompre la manœuvre de connexion et de désaccoupler les deux dispositifs de raccordement, ce qui peut entraîner une pollution des éléments d'étanchéité qui ne sont alors plus intégralement recouverts par les tiroirs de protection.

### OBJET DE L'INVENTION

L'invention a donc pour but de proposer un dispositif de raccordement aseptique d'un tube permettant d'obvier au moins en partie à l'inconvénient précité.

### RESUME DE L'INVENTION

A cet effet, l'invention propose un dispositif de raccordement aseptique d'un tube, comprenant :
- un corps comportant des moyens de couplage à un autre dispositif de raccordement et un embout tubulaire qui est agencé pour être engagé dans le tube, un canal s'étendant longitudinalement dans l'embout et débouchant dans un logement formé dans le corps et ouvert sur une surface dudit corps opposée à l'embout, le logement recevant un élément d'étanchéité ;
- un tiroir de protection de l'élément d'étanchéité, monté coulissant dans le corps parallèlement à ladite surface entre une position d'obturation du logement et une position de dégagement du logement ; et
- des moyens d'encliquetage à l'autre dispositif de raccordement, agencés pour empêcher, lors du couplage du dispositif de raccordement avec l'autre dispositif de raccordement, un désaccouplement dudit dispositif de raccordement et dudit autre dispositif de raccordement.

En empêchant le désaccouplement des dispositifs de raccordement, les moyens d'encliquetage permettent d'éviter la pollution des éléments d'étanchéité et donc de préserver la qualité aseptique du dispositif de raccordement.

Selon une caractéristique particulière, les moyens d'encliquetage sont agencés pour empêcher un désaccouplement du dispositif de raccordement avec l'autre dispositif de raccordement avant que le tiroir ne quitte sa position d'obturation lors du couplage dudit dispositif de raccordement avec ledit autre dispositif de raccordement.

Selon une autre caractéristique particulière, les moyens de couplage comprennent un élément d'enfichage mâle et un élément d'enfichage femelle destinés à coopérer respectivement avec un élément d'enfichage femelle et un élément d'enfichage mâle de l'autre dispositif de raccordement, le tiroir étant monté coulissant dans une cavité de l'élément d'enfichage femelle et pourvu d'une fente agencée pour recevoir l'élément d'enfichage mâle de l'autre dispositif de raccordement.

De manière particulière, l'élément d'enfichage mâle et l'élément d'enfichage femelle s'étendent orthogonalement à l'embout.

De manière particulière, l'élément d'enfichage mâle et le tiroir comprennent respectivement au moins une portion crantée et au moins une patte de retenue élastiquement déformable, la portion crantée et la patte de retenue formant les moyens d'encliquetage et étant agencés pour coopérer avec respectivement une patte de retenue et une portion crantée de l'autre dispositif de raccordement.

De manière particulière, la portion crantée comprend une succession de reliefs en dent de sapin.

De manière particulière, l'élément d'enfichage mâle et le tiroir comprennent respectivement deux portions crantées et deux pattes de retenue, les portions crantées et les pattes de retenue étant agencées symétriquement de part et d'autre d'un plan contenant un axe central de l'embout.

Selon une autre caractéristique particulière, le tiroir est agencé pour se déplacer de la position d'obturation à la position de dégagement sous l'effet d'un effort transversal destiné à être exercé par le corps de l'autre dispositif de raccordement lors du couplage du dispositif de raccordement avec ledit autre dispositif de raccordement.

Selon une autre caractéristique particulière, le dispositif comprend des moyens de verrouillage du tiroir en position d'obturation et en position de dégagement.

Selon une autre caractéristique particulière, les moyens de verrouillage sont agencés pour réaliser un son audible lorsque le tiroir rejoint la position de dégagement.

L'invention concerne également un ensemble de raccordement de deux tubes comprenant deux tels dispositifs de raccordement.

Avantageusement, les deux dispositifs de raccordement sont identiques.

### BREVE DESCRIPTION DES DESSINS

L'invention sera mieux comprise à la lumière de la description qui suit, laquelle est purement illustrative et non limitative, et doit être lue en regard des dessins annexés, parmi lesquels :
[Fig. 1] la figure 1 est une vue en perspective d'un dispositif de raccordement aseptique selon un mode de réalisation de l'invention, dans laquelle le tiroir est dans une position d'obturation ;
[Fig. 2] la figure 2 est une autre vue en perspective du dispositif de raccordement illustré à la figure 1 ;
[Fig. 3] la figure 3 est une vue analogue à celle de la figure 2, dans laquelle le tiroir est dans une position de dégagement ;
[Fig. 4] la figure 4 est une vue éclatée du dispositif de raccordement illustré aux figures 1 à 3 ;
[Fig. 5] la figure 5 est une vue éclatée analogue à celle de la figure 4, mais selon un angle de vue différent ; [Fig. 6] la figure 6 est une vue en coupe axiale du dispositif de raccordement illustré à la figure 1 ;
[Fig. 7A] la figure 7A est une vue en coupe axiale d'un ensemble de raccordement aseptique selon un mode de réalisation particulier de l'invention, avant connexion ; [Fig. 7B] la figure 7B est une vue en coupe transversale de l'ensemble de raccordement illustré à la figure 6, avant connexion ;
[Fig. 8A] la figure 8A est une vue analogue à la figure 7A pendant la connexion, avant un déplacement des tiroirs et une déformation des éléments d'étanchéité ;
[Fig. 8B] la figure 8B est une vue analogue à la figure 7B pendant la connexion, avant un déplacement des tiroirs et une déformation des éléments d'étanchéité ;
[Fig. 9A] la figure 9A est une vue analogue à la figure 7A pendant la connexion, avant un déplacement des tiroirs et au début d'une déformation des éléments d'étanchéité ;
[Fig. 9B] la figure 9B est une vue analogue à la figure 7B pendant la connexion, avant un déplacement des tiroirs et au début d'une déformation des éléments d'étanchéité ; [Fig. 10A] la figure 10A est une vue analogue à la figure 7A pendant la connexion, au début du déplacement des tiroirs ;
[Fig. 10B] la figure 10B est une vue analogue à la figure 7B pendant la connexion, au début du déplacement des tiroirs ;
[Fig. 11A] la figure 11A est une vue analogue à la figure 7A, après connexion ;
[Fig. 11B] la figure 11B est une vue analogue à la figure 7B, après connexion.

### DESCRIPTION DETAILLEE DE L'INVENTION

L'invention est ici décrite en application au raccordement d'un premier tube 100 stérile, formant un premier conduit de transport de fluide, à un deuxième tube 200 stérile formant un deuxième conduit de transport de fluide.

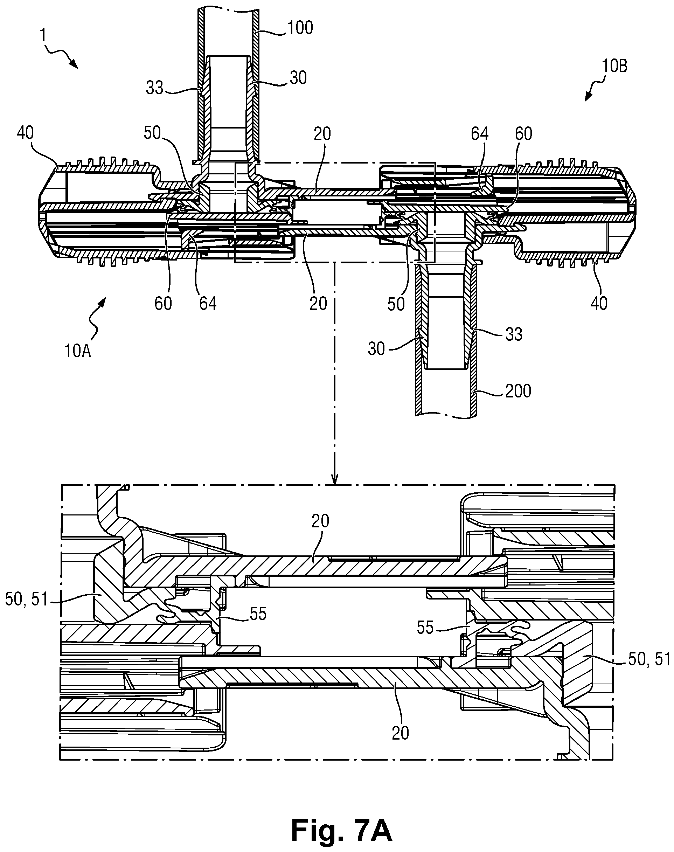

En référence aux figures 7A à 11B, un ensemble de raccordement fluidique généralement désigné en 1, selon un mode de réalisation particulier de l'invention, comprend deux dispositifs 10 de raccordement associés respectivement au premier tube 100 et au deuxième tube 200 pour réaliser ensemble une connexion aseptique entre ledit premier tube 100 et ledit deuxième tube 200. Les deux dispositifs 10 de raccordement sont identiques et sont distingués sur les figures par l'adjonction des lettres A, B.

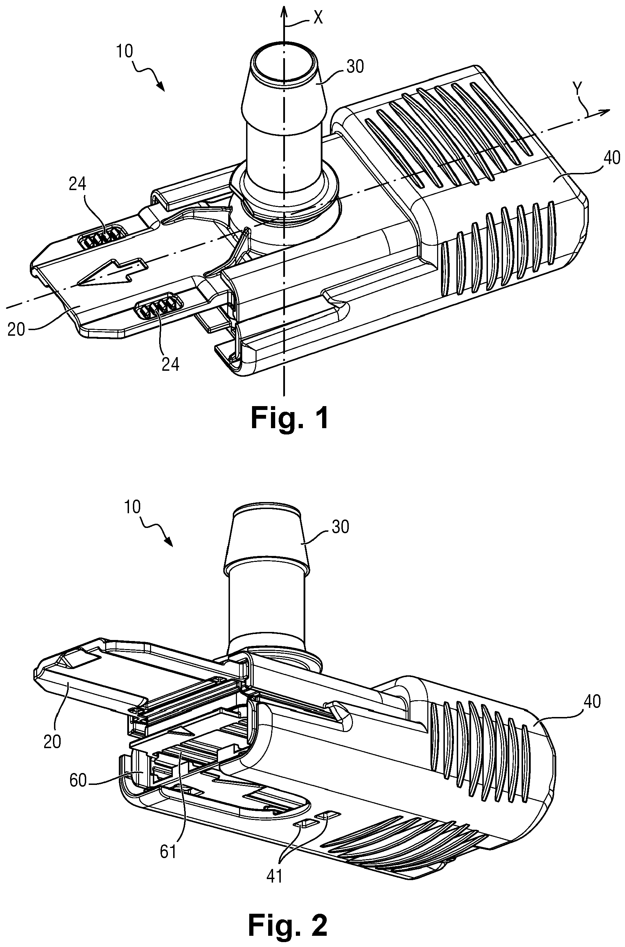

Comme illustré aux figures 1 à 6, chaque dispositif 10 comprend un corps réalisé ici en deux parties : une première partie comportant un élément d'enfichage mâle 20 pourvu d'un embout 30 tubulaire, et une seconde partie comportant un élément d'enfichage femelle 40 sur lequel est fixé l'élément d'enfichage mâle 20. L'élément d'enfichage mâle 20 et l'élément d'enfichage femelle 40 sont destinés à coopérer respectivement avec l'élément d'enfichage mâle 20 et l'élément d'enfichage femelle 40 de l'autre dispositif 10.

L'embout 30 s'étend selon un axe X, perpendiculairement en saillie d'une première surface supérieure 21.1 de l'élément d'enfichage mâle 20 qui est de forme globalement rectangulaire et qui s'étend selon un axe Y contenu dans un plan orthogonal à l'axe X. L'embout 30 délimite un canal 31 de passage de fluide débouchant sur une première surface inférieure 21.2 de l'élément d'enfichage mâle 20, opposée à la première surface supérieure 21.1.

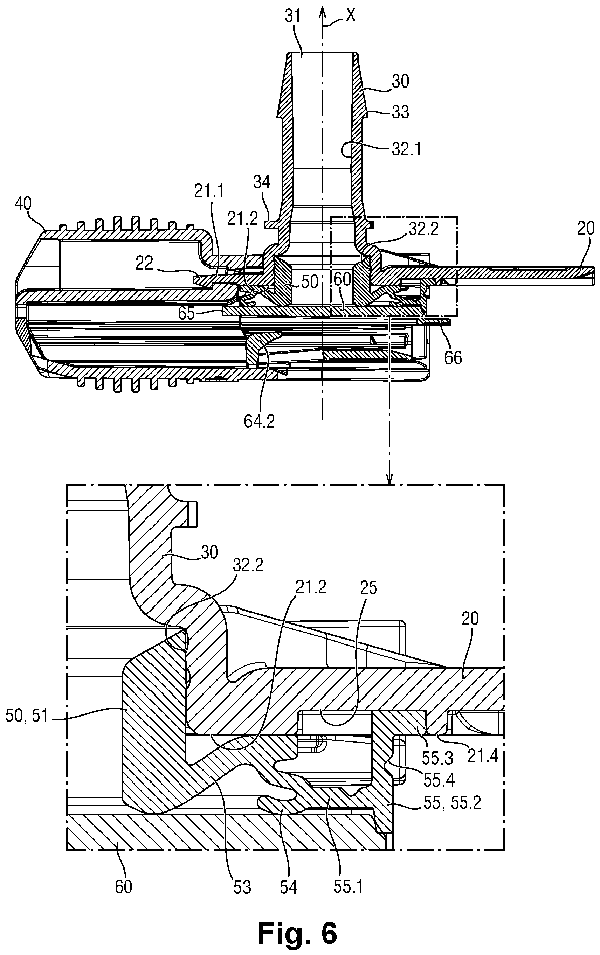

En référence à la figure 6, l'embout 30 comporte un premier tronçon d'extrémité délimitant intérieurement un premier alésage 32.1 qui débouche sur une extrémité libre de l'embout 30 et qui forme une première portion du canal 31. Le premier tronçon d'extrémité comprend extérieurement un relief 33 en dent de sapin et un collet 34 formant respectivement un moyen de son ancrage dans le tube 100, 200 et une butée à son enfoncement dans ledit tube 100, 200.

L'embout 30 comporte, à l'opposé du premier tronçon d'extrémité, un deuxième tronçon d'extrémité délimitant intérieurement un deuxième alésage 32.2 coaxial au premier alésage 32.1. Le deuxième alésage 32.2 débouche sur la première surface inférieure 21.2 de l'élément d'enfichage mâle 20 et forme une deuxième portion du canal 31. Le diamètre du deuxième alésage 32.2 est supérieur à celui du premier alésage 32.1.

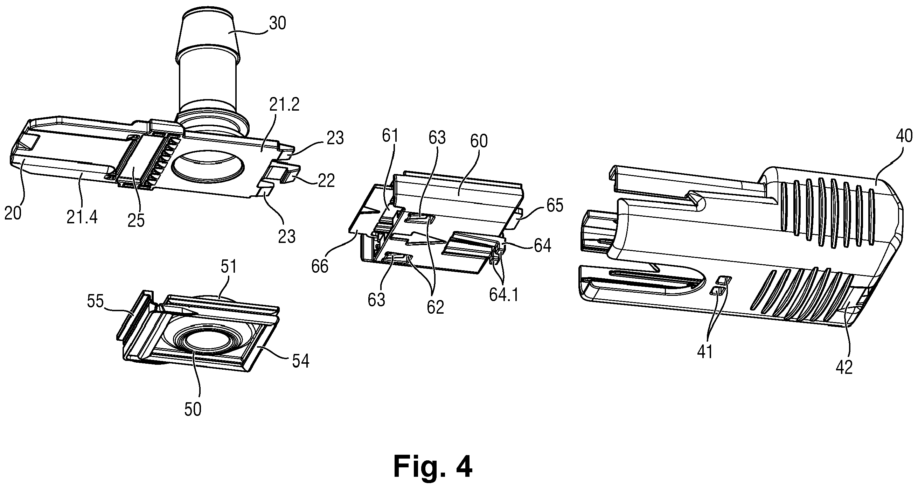

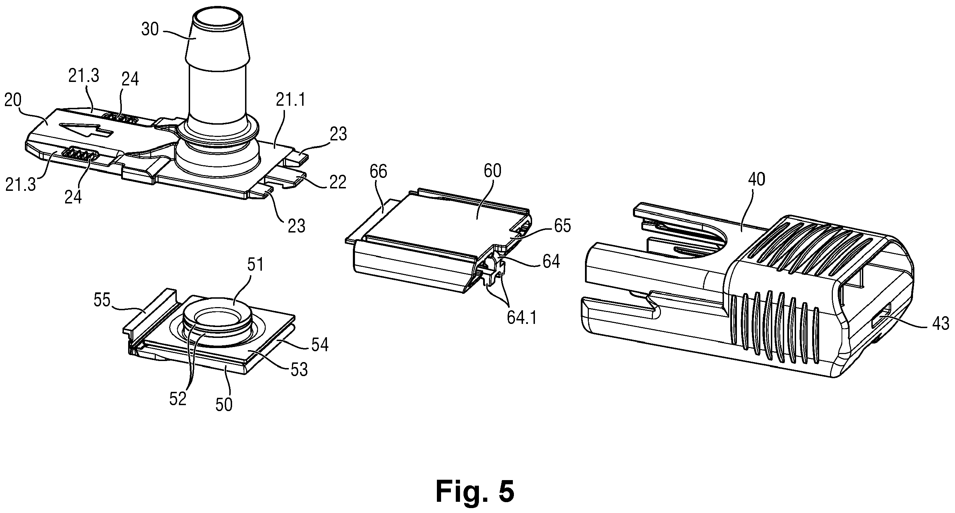

En référence aux figures 4 et 5, l'élément d'enfichage mâle 20 comprend une première extrémité au voisinage de laquelle s'étend l'embout 30. La première extrémité est pourvue d'une patte de fixation 22 centrale et de deux plots de centrage 23 latéraux assurant la fixation dudit élément d'enfichage mâle 20 sur l'élément d'enfichage femelle 40.

L'élément d'enfichage mâle 20 comprend également une deuxième extrémité, opposée à la première extrémité, formant une extrémité libre dudit élément d'enfichage mâle 20. La deuxième extrémité est pourvue de deux portions crantées 24 s'étendant symétriquement de part et d'autre du plan XY définis par les axes X, Y (figures 1 et 5). Chacune des portions crantées 24 comprend une succession de quatre reliefs 24.1, 24.2, 24.3, 24.4 en dent de sapin s'étendant en saillie d'une deuxième surface supérieure 21.3 de l'élément d'enfichage mâle 20 pour former, comme on le verra plus loin, des moyens d'encliquetage de l'élément d'enfichage mâle 20 dans le tiroir 60 de l'autre dispositif 10. Les reliefs 24.1, 24.2, 24.3, 24.4 sont identiques et alignés suivant un axe parallèle à l'axe X.

L'élément d'enfichage mâle 20 comprend également un évidement 25 rectangulaire ouvert sur une deuxième surface inférieure 21.4 de l'élément d'enfichage mâle 20, opposée à la deuxième surface supérieure 21.3 (figure 4). L'évidement s'étend suivant un axe orthogonal aux axes X, Y.

Le dispositif 10 comprend également un élément d'étanchéité 50 comportant un insert 51 tubulaire qui est reçu dans le deuxième alésage 32.2 de l'embout 30 et qui délimite une section de passage de fluide globalement constante. L'insert 51 a une première extrémité en regard d'un fond du deuxième alésage 32.2, et une deuxième extrémité en saillie vers l'extérieur dudit deuxième alésage 32.2, au-delà de la première surface inférieure 21.2 de l'élément d'enfichage mâle 20. L'insert 51 est pourvu extérieurement de deux reliefs annulaires 52 en saillie radiale pour être en contact étanche avec le deuxième alésage 32.2. L'insert 51 est également pourvu extérieurement d'une collerette 53 ayant une face arrière en appui contre la première surface inférieure 21.2 de l'élément d'enfichage mâle 20. Le deuxième alésage 32.2 de l'embout 30 et la première surface inférieure 21.2 de l'élément d'enfichage mâle 20 délimitent ensemble un logement recevant l'élément d'étanchéité 50.

La collerette 53 est élastiquement déformable et s'étend légèrement en arrière de la deuxième extrémité de l'insert 51. Une face avant de la collerette 53, opposée à la face arrière, forme une face frontale de l'élément d'étanchéité 50 de laquelle s'étend en saillie la deuxième extrémité de l'insert 51 de telle manière que ladite deuxième extrémité puisse venir en contact étanche avec la deuxième extrémité de l'élément d'étanchéité 50 de l'autre dispositif 10. La collerette 53 a un pourtour extérieur, de forme carrée, comportant un bourrelet 54 qui s'étend en saillie de la face avant de ladite collerette 53 et qui est destiné à être en contact étanche avec celui de l'élément d'étanchéité 50 de l'autre dispositif 10.

L'élément d'étanchéité 50 comporte également une lèvre 55 de protection, en forme de marche d'escalier, s'étendant depuis un flanc latéral externe du bourrelet 54. La lèvre 55 est élastiquement déformable et comprend :
- une première portion 55.1 plane de forme rectangulaire, s'étendant depuis le flanc latéral du bourrelet 54 dans un plan orthogonal à l'axe X, suivant un axe parallèle à l'axe Y ;
- une deuxième portion 55.2 plane de forme rectangulaire, reliée à la première portion 55.1 et s'étendant depuis une extrémité distale de ladite première portion 55.1 dans un plan orthogonal à l'axe Y, suivant un axe parallèle à l'axe X ; et
- une troisième portion 55.3 plane de forme rectangulaire, reliée à la deuxième portion 55.2 et s'étendant depuis une extrémité distale de ladite deuxième portion 55.2 dans un plan orthogonal à l'axe X, suivant un axe parallèle à l'axe Y.

La deuxième portion 55.2 comprend une rainure 55.4 droite qui est ouverte sur une face frontale de la lèvre 55 et qui s'étend suivant un axe orthogonal aux axes X, Y. La troisième portion 55.3 vient se loger dans l'évidement 25 de l'élément d'enfichage mâle 20 et a une extrémité libre sensiblement en appui contre une paroi latérale dudit évidement 25.

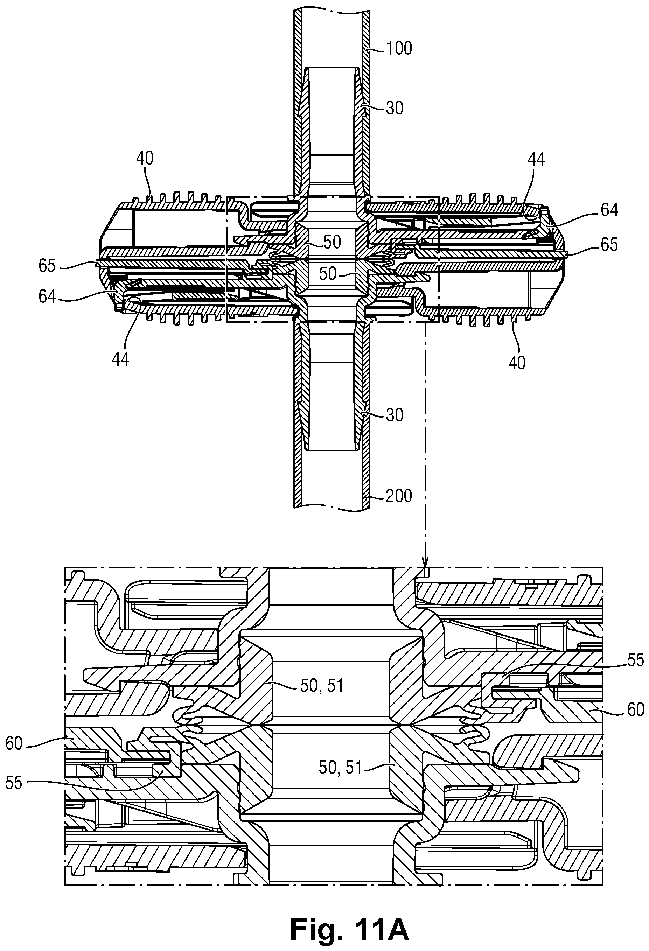

L'insert 51 de l'élément d'étanchéité 50 est monté mobile axialement dans le deuxième alésage 32.2 de l'embout 30 entre une première position dite « avancée » légèrement en saillie du bourrelet 54 et dans laquelle la première extrémité de l'insert 51 est éloigné du fond du deuxième alésage 32.2 (figure 11A), et une deuxième position dite « reculée » en retrait de la première position et dans laquelle la première extrémité de l'insert 51 est en contact avec ledit fond du deuxième alésage 32.2 (figures 6 et 7A). L'insert 51 est rappelé dans la position avancée par la collerette 53 qui passe d'un état déformé à un état de repos.

On notera que la première extrémité de l'insert 51 a ici une surface tronconique pour amener le fluide circulant dans le canal 31 à exercer sur ladite première extrémité un effort tendant à amener l'insert 51 en position avancée.

L'insert 51 est agencé pour être amené de sa position avancée à sa position reculée sous l'effet d'un effort transversal exercé sur la deuxième extrémité de l'insert 51 par un tiroir 60 de protection, de forme globalement parallélépipédique, s'étendant parallèlement à la première surface inférieure 21.2.

Le tiroir 60 comprend une fente 61 ouverte sur une face avant dudit tiroir 60 et destinée à recevoir l'extrémité libre de l'élément d'enfichage mâle 20 de l'autre dispositif 10.

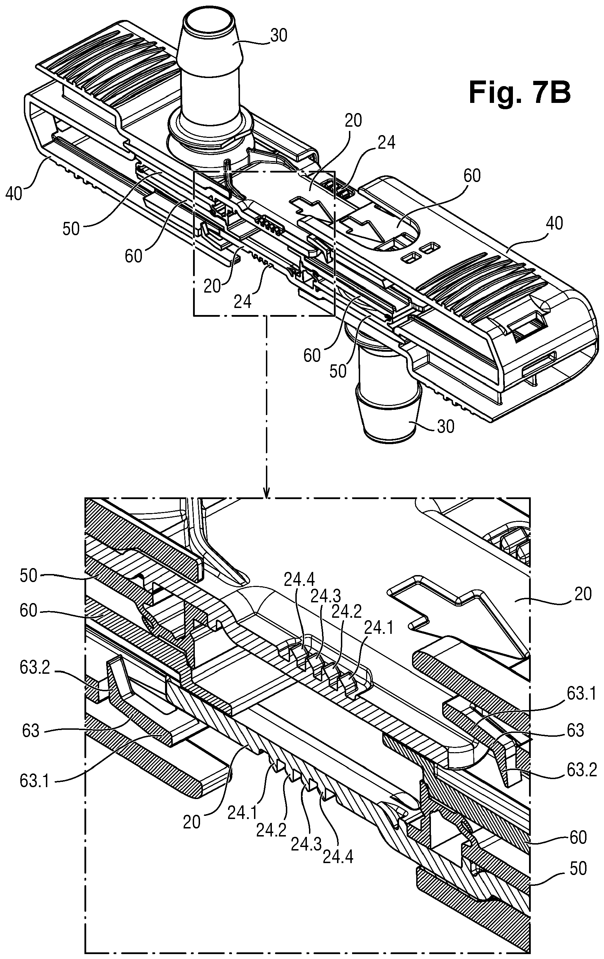

Une face inférieure du tiroir 60 comporte deux ouvertures 62 identiques s'étendant symétriquement de part et d'autre du plan XY, chacune au voisinage d'un bord latéral de la face inférieure et à proximité de la face avant du tiroir 60 (figure 4). A l'intérieur de chacune des ouvertures 62 s'étend une patte de retenue 63 destinée à coopérer avec les reliefs 24.1, 24.2, 24.3, 24.4 des portions crantées 24 pour former les moyens d'encliquetage de l'élément d'enfichage mâle 20 de l'autre dispositif 10 dans le tiroir 60. Chaque patte de retenue 63 a une portion sensiblement plane 63.1 dont une extrémité est solidaire d'un bord de l'ouverture 62 correspondante, et une portion inclinée 63.2 s'étendant dans le prolongement de la portion plane 63.1 et dont une extrémité libre est dans une position en saillie de la fente 61 du tiroir 60 (figure 7B) .

Les pattes de retenue 63 sont identiques et sont élastiquement déformables entre un état de repos dans lequel leurs extrémités libres délimitent une hauteur de section de passage inférieure à une hauteur de l'élément d'enfichage mâle 20 de sorte que les pattes de retenue 63 s'opposent à l'engagement de l'élément d'enfichage mâle 20 de l'autre dispositif 10 dans la fente 61 du tiroir 60, et un état déformé dans lequel leurs extrémités libres sont dans une position escamotée dans laquelle elles délimitent une hauteur de section de passage supérieure à une hauteur de l'élément d'enfichage mâle 20.

La face inférieure du tiroir 60 comprend également deux rainures longitudinales débouchant sur une face arrière du tiroir 60 pour délimiter une patte d'ancrage 64. La patte d'ancrage 64 a une extrémité solidaire du reste de la paroi supérieure et une extrémité opposée qui est libre et pourvue de deux tenons 64.1 qui sont dans une position en saillie de la face inférieure pour former, comme on le verra plus loin, des moyens de verrouillage du tiroir 60 en position. L'extrémité libre de la patte d'ancrage 64 est également pourvue d'un bec de commande 64.2 s'étendant en saillie de la fente 61 du tiroir 60 (figure 7A). Le bec de commande 64.2 comprend une surface plane inclinée formant une première rampe de soulèvement de la patte d'ancrage 64 destinée à coopérer avec l'extrémité libre de l'élément d'enfichage mâle 20 de l'autre dispositif 10.

La patte d'ancrage 64 est élastiquement déformable entre un état de repos dans lequel les tenons 64.1 s'étendent en saillie de la face inférieure du tiroir 60, et un état déformé dans lequel lesdits tenons 64.1 s'étendent en retrait de ladite face inférieure du tiroir 60.

Comme on le verra plus loin, la patte d'ancrage 64 est agencée pour passer de son état de repos à son état déformé sous l'effet d'un effort transversal exercé par l'extrémité libre de l'élément d'enfichage mâle 20 de l'autre dispositif 10 sur la surface inclinée du bec de commande 64.2.

En référence aux figures 4 et 5, le tiroir 60 comprend en outre :
- un premier appendice 65 s'étendant en saillie de la face arrière dudit tiroir 60 pour former, comme on le verra plus loin, un témoin d'enfoncement du tiroir 60 ; et
- un deuxième appendice 66 s'étendant en saillie de la face avant dudit tiroir 60 pour entrer en contact, comme on le verra plus loin, avec la deuxième portion 55.2 de la lèvre 55 de l'élément d'étanchéité 50.

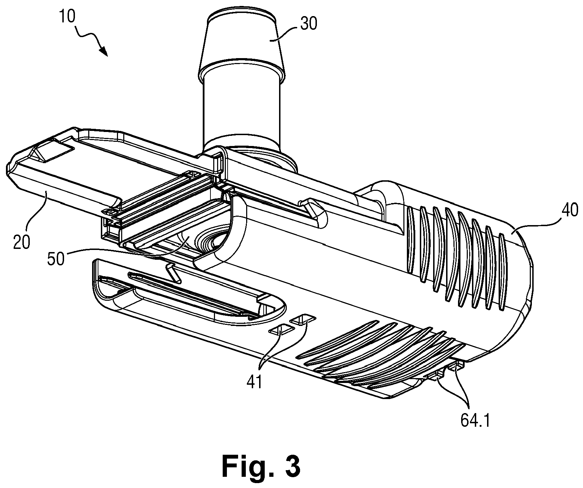

Le tiroir 60 est monté coulissant selon l'axe X dans une cavité ouverte ménagée dans l'élément d'enfichage femelle 40, entre :
- une première position dite d'obturation du logement recevant l'élément d'étanchéité 50, dans laquelle le tiroir 60 s'étend en regard dudit élément d'étanchéité 50 en étant en contact étanche avec le bourrelet 54 et une extrémité libre de la deuxième portion 55.2 de la lèvre 55 de l'élément d'étanchéité 50, et en exerçant sur la deuxième extrémité de l'insert 51 un effort axial de sorte que ledit insert 51 est en position reculée (figures 2, 6 et 7A), et
- une deuxième position dite de dégagement du logement recevant l'élément d'étanchéité 50, dans laquelle le tiroir 60 s'étend en retrait de l'élément d'étanchéité 50 sorte que l'insert 51 est en position avancée (figures 3 et 11A).

Lorsque le tiroir 60 est dans sa position d'obturation, les tenons 64.1 de la patte d'ancrage 64 sont reçus chacun dans un premier trou 41 ménagé dans l'élément d'enfichage femelle 40 de sorte que lesdits tenons 64.1 s'opposent au déplacement du tiroir 60 vers la position de dégagement.

Lorsque le tiroir 60 est dans sa position de dégagement, les tenons 64.1 de la patte d'ancrage 64 sont reçus dans un même deuxième trou 42 ménagé dans l'élément d'enfichage femelle 40 de sorte que lesdits tenons 64.1 s'opposent au déplacement du tiroir 60 vers la position d'obturation, et le premier appendice 65 du tiroir 60 est reçu dans un troisième trou 43 ménagé dans l'élément d'enfichage femelle 40 de sorte que ledit premier appendice 65 est parfaitement visible lorsque ledit tiroir 60 est dans sa position de dégagement.

Les tenons 64.1 de la patte d'ancrage 64 forment ainsi des moyens de verrouillage du tiroir 60 dans sa position d'obturation et dans sa position de dégagement, et le premier appendice 65 forme un témoin d'enfoncement visuel du tiroir 60 dans sa position de dégagement.

Comme on le verra plus loin, le tiroir 60 est agencé pour être amené de sa position d'obturation à sa position de dégagement sous l'effet d'un effort transversal exercé par l'extrémité libre de l'élément d'enfichage mâle 20 de l'autre dispositif 10 sur la surface inclinée du bec de commande 64.2 de la patte d'ancrage 64.

Afin d'éviter toute détérioration et contamination de l'embout 30 lors du stockage et du transport du dispositif 10, un capot de protection (non représenté) est monté de manière amovible sur ledit embout 30. Le capot peut par exemple être agencé pour se clipper (emboîtement élastique) sur le relief 33 en dent de sapin.

On notera que l'ensemble du dispositif 10 est stérilisé en usine afin d'être exempt de toute contamination avant son stockage.

Le fonctionnement de l'ensemble de raccordement 1 va maintenant être détaillé au regard des figures 7A à 11B (pour plus de clarté, les deux dispositifs 10 sont distingués l'un de l'autre par les lettres A et B).

Le capot de protection du premier dispositif 10A est tout d'abord retiré de l'embout 30 rendant notamment visible le relief 33 en dent de sapin et le collet 34. L'embout 30 du premier dispositif 10A est alors emmanché dans le premier tube 100 jusqu'à ce que celui-ci vienne en butée contre le collet 34 dudit premier dispositif 10A. De même, le capot de protection du deuxième dispositif 10B est retiré de l'embout 30 qui est alors emmanché dans le deuxième tube 200 jusqu'à ce que celui-ci vienne en butée contre le collet 34 dudit deuxième dispositif 10B, de sorte que les premier et deuxième dispositifs 10A, 10B sont respectivement ancrés dans les premier et deuxième tubes 100, 200.

Les premier et deuxième dispositifs 10A, 10B sont ensuite présentés en regard l'un de l'autre, le premier dispositif 10A étant tourné axialement de 180 degrés par rapport au deuxième dispositif 10B de sorte que l'élément d'enfichage mâle 20 et le tiroir 60 du premier dispositif 10A sont respectivement en regard du tiroir 60 et de l'élément d'enfichage mâle 20 du deuxième dispositif 10B, les tiroirs 60 des premiers et deuxième dispositifs 10A, 10B étant verrouillés en position de d'obturation via les pattes d'ancrage 64 desdits tiroirs 60 (figures 7A et 7B).

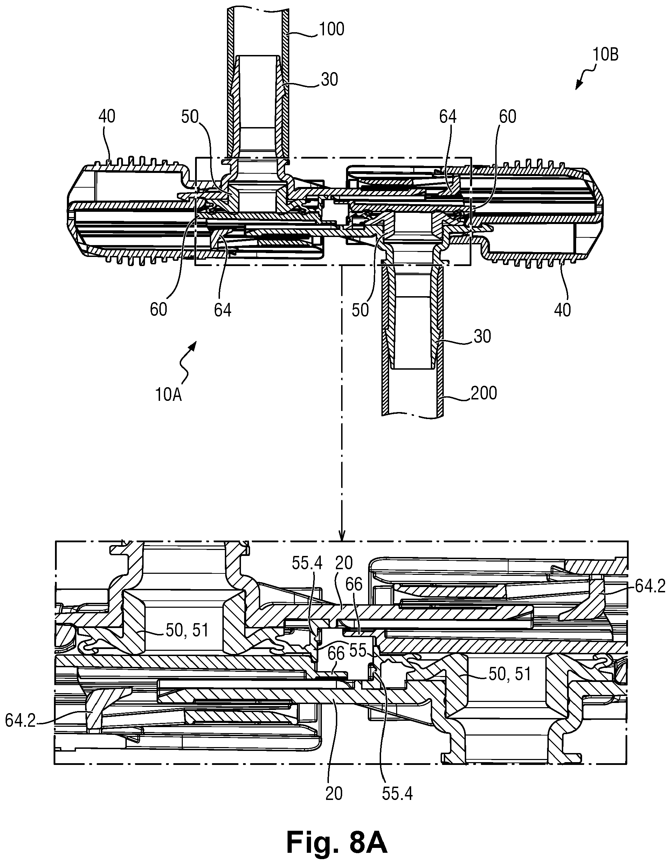

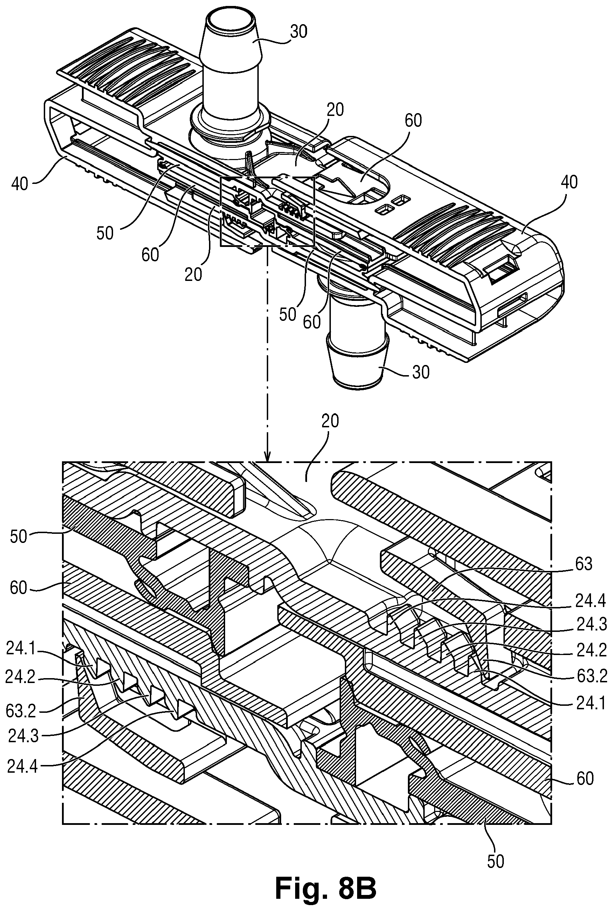

Dans un premier temps, les premier et deuxième dispositifs 10A, 10B sont rapprochés transversalement l'un de l'autre, c'est-à-dire selon l'axe Y, jusqu'à ce que les pattes de retenue 63 des tiroirs 60 des premier et deuxième dispositifs 10A, 10B soient en appui contre les premiers reliefs 24.1 des portions crantées 24 desdits premier et deuxième dispositifs 10A, 10B. Le tiroir 60 du premier dispositif 10A reçoit alors une portion de l'élément d'enfichage mâle 20 du deuxième dispositif 10B et le tiroir 60 du deuxième dispositif 10B reçoit une portion de l'élément d'enfichage mâle 20 du premier dispositif 10A (figures 8A et 8B).

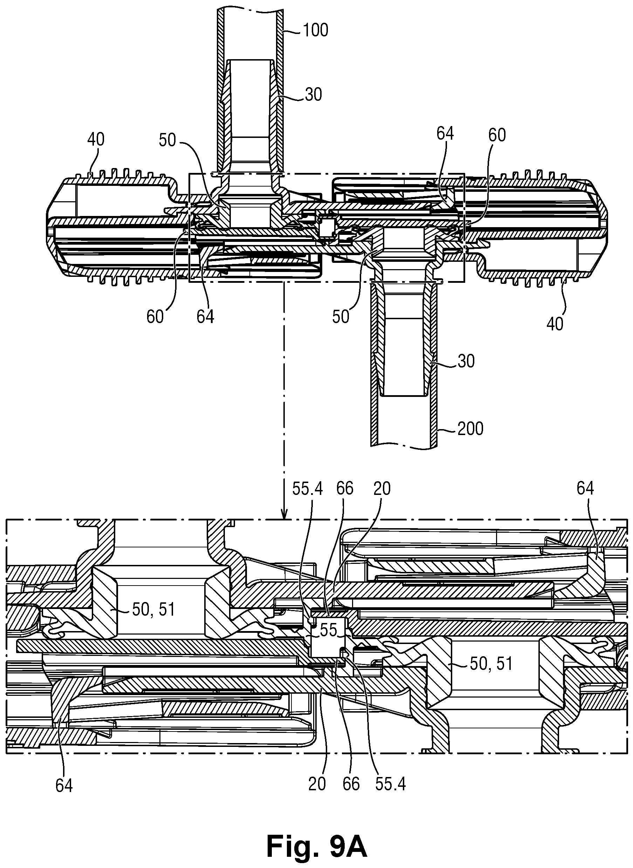

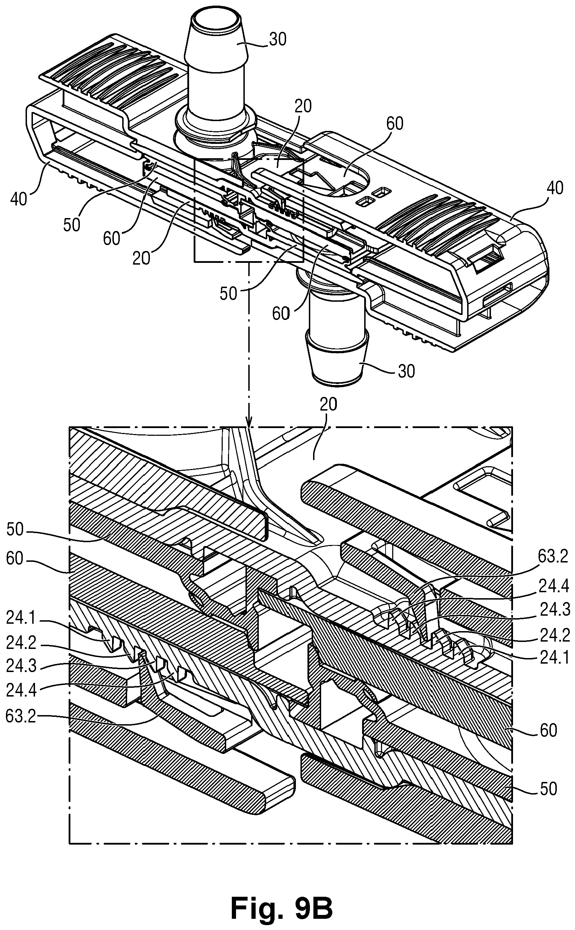

En poursuivant le rapprochement des premier et deuxième dispositifs 10A, 10B jusqu'à ce que les deuxièmes appendices 66 des tiroirs 60 entrent en contact avec les deuxièmes portions 55.2 des lèvres 55 des éléments d'étanchéité 50, les pattes de retenue 63 des tiroirs 60 s'encliquètent de manière audible sur les premiers reliefs 24.1 et les deuxièmes reliefs 24.2 des portions crantées 24 des éléments d'enfichage mâle 20, de sorte que lesdits éléments d'enfichage mâle 20 ne peuvent plus être désengagés des tiroirs 60 (figures 9A et 9B). Les premier et deuxième dispositifs 10A, 10B deviennent alors solidaires l'un de l'autre et ne peuvent plus être séparés.

On comprendra que l'on entend ici par encliqueter le fait que les pattes de retenue 63 des tiroirs 60 s'engagent et s'emboîtent élastiquement sur les premiers reliefs 24.1 et les deuxièmes reliefs 24.2 des portions crantées 24 des éléments d'enfichage mâle 20. Autrement dit, lesdites pattes de retenue 63 se clipsent sur lesdits premiers et deuxièmes reliefs 24.1, 24.2.

Dans un deuxième temps, les premier et deuxième dispositifs 10A, 10B continuent d'être rapprochés l'un de l'autre jusqu'à ce que les extrémités libres des éléments d'enfichage mâle 20 soient en contact avec les surfaces inclinées des becs de commande 64.2 des patte d'ancrage 64 des tiroirs 60, ce qui entraîne un encliquetage audible des pattes de retenue 63 des tiroirs 60 sur les troisièmes reliefs 24.3 des portions crantées 24, et une déformation des lèvres 55 via les deuxièmes appendices 66 des tiroirs qui exercent sur les deuxièmes portions 55.2 des lèvres 55 un effort axial tendant à décoller lesdites deuxièmes portions 55.2 des faces avant des tiroirs 60.

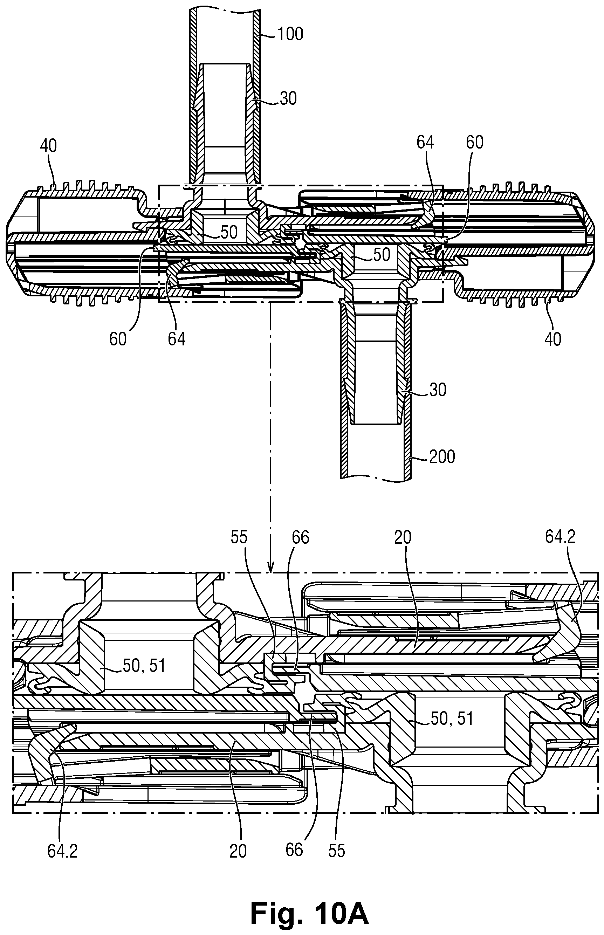

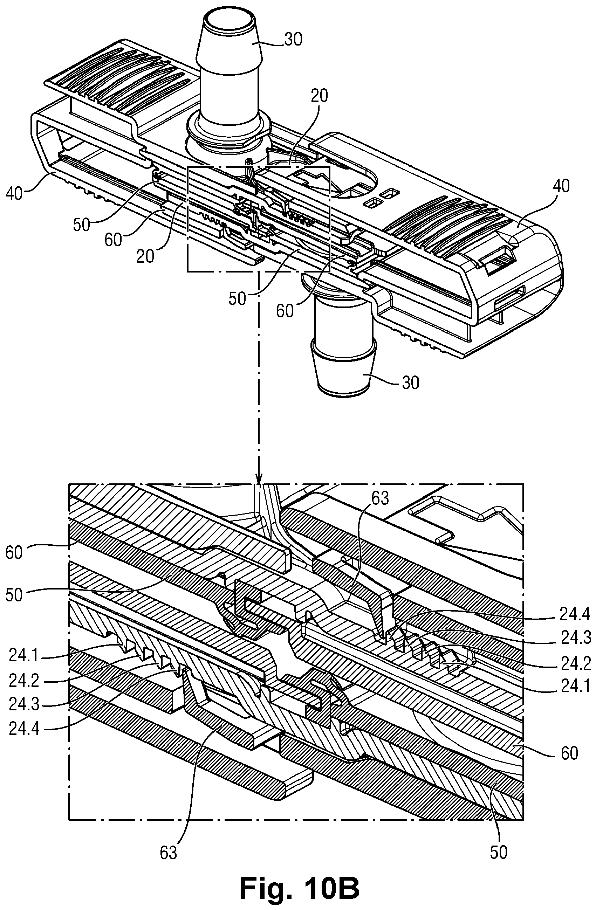

En poursuivant le rapprochement des premier et deuxième dispositifs 10A, 10B jusqu'à ce que les pattes de retenue 63 des tiroirs 60 s'encliquètent de manière audible sur les quatrièmes reliefs 24.4 des portions crantées 24 des éléments d'enfichage mâle 20, les extrémités libres des éléments d'enfichage mâle 20 exercent sur les surfaces inclinées des becs de commande 64.2 un effort transversal entraînant un escamotage desdites pattes d'ancrage 64 qui passent de l'état de repos à un état intermédiaire dans lequel les tenons 64.1 des pattes d'ancrage 64 s'étendent légèrement en retrait de la face inférieure du tiroir 60, l'état intermédiaire étant agencé entre ledit état de repos et l'état déformé (figure 10A, 10B). Les tiroirs 60 sont alors libres de se déplacer de leur position d'obturation à leur position de dégagement.

Dans un troisième temps, les premier et deuxième dispositifs 10A, 10B continuent d'être rapprochés l'un de l'autre, ce qui entraîne un déplacement des tiroirs 60 de leur position d'obturation vers leur position de dégagement, les extrémités libres des éléments d'enfichage continuant d'exercer un effort transversal sur les surfaces inclinées des becs de commande 64.2 des pattes d'ancrage 64.

En poursuivant le rapprochement des premier et deuxième dispositifs 10A, 10B, les tiroirs 60 finissent par ne plus masquer les éléments d'étanchéité 50 et n'exercent alors plus aucun effort sur les inserts 51 qui passent donc de leur position reculée à leur position avancée.

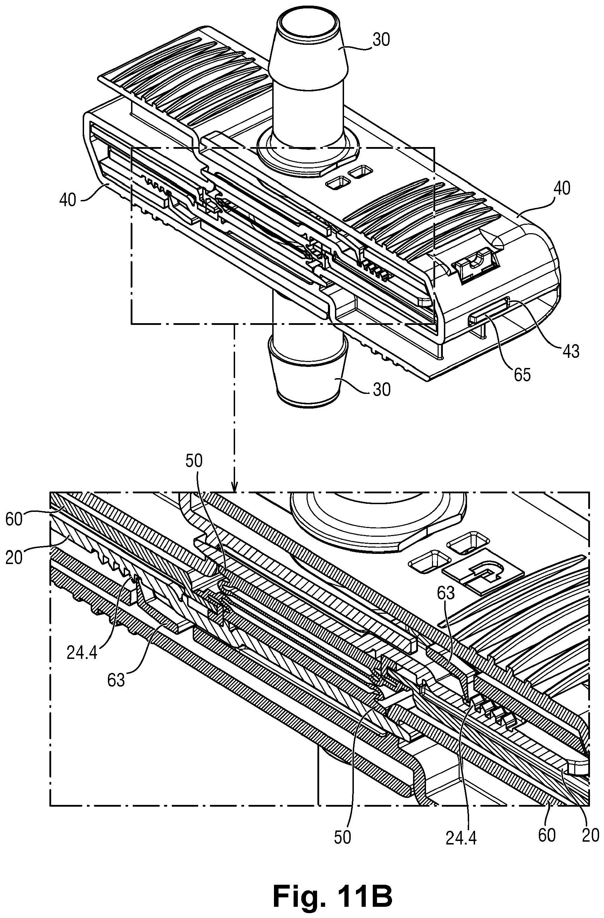

Dans un quatrième temps, les premier et deuxième dispositifs 10A, 10B continuent d'être rapprochés l'un de l'autre jusqu'à ce que l'embout 30 du premier du dispositif 10A s'étende coaxialement à l'embout 30 du deuxième dispositif 10B et que les tiroirs 60 rejoignent leur position de dégagement (figure 11A et 11B). Les inserts 51 des premier et deuxième dispositifs 10A, 10B exercent alors l'un sur l'autre un effort axial assurant le retour desdits inserts 51 en position reculée et une mise en contact étanche de ceux-ci, et le bourrelet 54 du premier dispositif 10A est en contact étanche avec le bourrelet 54 du deuxième dispositif 10B.

Dans le même temps, les tenons 64.1 des pattes d'ancrage 64 glissent sur des surfaces internes 44 des éléments d'enfichage femelles 40 formant des deuxièmes rampes de soulèvement des pattes d'ancrage 64 qui passent alors de l'état intermédiaire à l'état déformé avant de repasser à l'état intermédiaire en effectuant un clic audible engendré par l'engagement élastique des tenons 64.1 dans les deuxièmes trous 42 des éléments d'enfichage femelles 40. Les tiroirs 60 sont alors verrouillés en position de dégagement. Le clic audible fait office de témoin sonore garantissant que les tiroirs 60 sont en position de dégagement et donc que l'insert 51 et le bourrelet 54 de l'élément d'étanchéité 50 du premier dispositif 10A sont respectivement en contact étanche avec l'insert 51 et le bourrelet 54 de l'élément d'étanchéité 50 du deuxième dispositif 10B.

Dans le même temps également, les premiers appendices 65 des tiroirs 60 sont reçus dans les troisièmes trous 43 des éléments d'enfichage femelles 40 de sorte que lesdits premiers appendices 65 font office de témoins visuels garantissant que les tiroirs 60 sont en position de dégagement.

On notera qu'en étant encliquetées sur les quatrièmes reliefs 24.4 des portions crantées 24 des éléments d'enfichage mâle 20, les pattes d'ancrage 64 des tiroirs 60 empêchent tout dégagement des éléments d'enfichage mâles 20 des tiroirs 60 qui sont verrouillés en position de dégagement.

On parle alors de couplage des premier et deuxième dispositifs 10A, 10B qui forment ainsi ensemble un conduit étanche pour le passage d'un fluide entre le premier tube 100 et le deuxième tube 200.

On notera que le décollement des lèvres 55 de la face avant des tiroirs 60 permet d'éviter tout contact entre l'extrémité libre des deuxièmes portions 55.2 des lèvres 55 et les faces supérieures des tiroirs 60, et donc de limiter la contamination desdits éléments d'étanchéité 50.

Bien entendu, l'invention n'est pas limitée au mode de réalisation décrit mais englobe toute variante entrant dans le champ de l'invention telle que définie par les revendications.

Bien que le corps soit ici réalisé en deux parties, il peut aussi être réalisé en une seule partie ou en au moins trois parties.

Le nombre de reliefs 24.1, 24.2, 24.3, 24.4 des portions crantées 24 peut être inférieur ou supérieur à quatre.

Les portions crantées 24 peuvent s'étendre sur une longueur inférieure ou supérieure à celle illustrée à la figure 1, et par exemple couvrir au minimum le moment où les deuxièmes appendices 66 des tiroirs 60 sont reçus dans les rainures 55.4 des lèvres des élément d'étanchéité 50 (figures 9A et 9B) et le moment où le tiroir a rejoint sa position de dégagement (figures 11A et 11B).

Les pattes de retenue 63 et les portions crantées 24 peuvent être agencées sur respectivement des faces latérales du tiroir 60 et des faces latérales des éléments d'enfichage mâle 20.

Les pattes de retenue 63 peuvent être rigides, la paroi du tiroir 60 portant lesdites pattes de retenue 63 étant alors élastiquement déformable de manière à permettre l'encliquetage de l'élément d'enfichage mâle 20 dans le tiroir 60.

Les pattes de retenue 63 peuvent être portées par l'élément d'enfichage mâle 20 et les portions crantées 24 par les tiroirs 60.

Bien que la patte d'ancrage 64 soit ici soulevée via l'effort transversal exercé par l'extrémité libre de l'élément d'enfichage mâle 20 de l'autre dispositif sur la surface inclinée du bec de commande 64.2, tout autre moyen de soulèvement peut être utilisé pour amener la patte d'ancrage 64 à l'état déformé.

La patte d'ancrage 64 peut être agencée de manière à pouvoir commander son passage de l'état de repos à l'état déformé indépendamment de la position du tiroir 60.

Le tiroir 60 peut être agencé pour se déplacer de la position d'obturation à la position de dégagement sous l'effet d'un effort transversal exercé par l'élément d'étanchéité 50 de l'autre dispositif lors du raccordement entre eux des deux dispositifs de raccordement.

Le tiroir 60 peut être agencé pour se déplacer de la position d'obturation à la position de dégagement sous l'effet d'un effort transversal exercé par l'élément d'enfichage femelle 40 de l'autre dispositif de raccordement lors du raccordement entre eux des deux dispositifs de raccordement.

Bien que l'ensemble de raccordement comprenne ici deux dispositifs 10A, 10B identiques, il peut aussi comprendre deux dispositifs différents.

Bien que l'ancrage des tubes 100, 200 sur les dispositifs 10A, 10B soit ici réalisé par un relief en dent de sapin, il peut aussi être réalisé par tout autre moyen tel qu'un jonc, une rondelle extérieurement dentée ou des bras élastiquement déformables.

Bien que le premier appendice 65 forme ici un témoin visuel d'enfoncement du tiroir 60 dans sa position de dégagement, il peut aussi former un témoin tactile d'enfoncement dudit tiroir 60, par exemple en s'étendant en saillie de l'élément d'enfichage femelle 40 lorsque le tiroir 60 est en position de dégagement.

## Revendications

1. Dispositif (10) de raccordement aseptique d'un tube (100, 200), comprenant :
- un corps (20, 30, 40) comportant des moyens de couplage (20, 40) à un autre dispositif (10) de raccordement et un embout (30) tubulaire qui est agencé pour être engagé dans le tube, un canal (31) s'étendant longitudinalement dans l'embout et débouchant dans un logement formé dans le corps et ouvert sur une surface dudit corps opposée à l'embout, le logement recevant un élément d'étanchéité (50) ;
- un tiroir (60) de protection de l'élément d'étanchéité, monté coulissant dans le corps parallèlement à ladite surface entre une position d'obturation du logement et une position de dégagement du logement ; et
- des moyens d'encliquetage (24, 63) à l'autre dispositif de raccordement, agencés pour empêcher, avant que le tiroir ne quitte sa position d'obturation lors du couplage du dispositif de raccordement avec l'autre dispositif de raccordement, un désaccouplement dudit dispositif de raccordement et dudit autre dispositif de raccordement.

2. Dispositif (10) de raccordement selon la revendication 1, dans lequel les moyens de couplage comprennent un élément d'enfichage mâle (20) et un élément d'enfichage femelle (40) destinés à coopérer respectivement avec un élément d'enfichage femelle et un élément d'enfichage mâle de l'autre dispositif de raccordement, le tiroir (60) étant monté coulissant dans une cavité de l'élément d'enfichage femelle et pourvu d'une fente (61) agencée pour recevoir l'élément d'enfichage mâle de l'autre dispositif (10) de raccordement.

3. Dispositif (10) de raccordement selon la revendication 2, dans lequel l'élément d'enfichage mâle (20) et l'élément d'enfichage femelle (40) s'étendent orthogonalement à l'embout (30).

4. Dispositif (10) de raccordement selon la revendication 2 ou 3, dans lequel l'élément d'enfichage mâle (20) et le tiroir (60) comprennent respectivement au moins une portion crantée (24) et au moins une patte de retenue (63) élastiquement déformable, la portion crantée et la patte de retenue formant les moyens d'encliquetage et étant agencés pour coopérer avec respectivement une patte de retenue et une portion crantée de l'autre dispositif de raccordement.

5. Dispositif (10) de raccordement selon la revendication 4, dans lequel la portion crantée (24) comprend une succession de reliefs (24.1, 24.2, 24.3, 24.4) en dent de sapin.

6. Dispositif (10) de raccordement selon la revendication 4 ou 5, dans lequel l'élément d'enfichage mâle (20) et le tiroir (60) comprennent respectivement deux portions crantées (24) et deux pattes de retenue (63), les portions crantées et les pattes de retenue étant agencées symétriquement de part et d'autre d'un plan contenant un axe (X) central de l'embout (30).

7. Dispositif (10) de raccordement selon l'une quelconque des revendications précédentes, dans lequel le tiroir (60) est agencé pour se déplacer de la position d'obturation à la position de dégagement sous l'effet d'un effort transversal destiné à être exercé par le corps (20, 30, 40) de l'autre dispositif de raccordement lors du couplage du dispositif de raccordement avec ledit autre dispositif de raccordement.

8. Dispositif (10) de raccordement selon l'une quelconque des revendications précédentes, comprenant des moyens de verrouillage (64) du tiroir (60) en position d'obturation et en position de dégagement.

9. Dispositif (10) de raccordement selon la revendication 8, dans lequel les moyens de verrouillage sont agencés pour réaliser un son audible lorsque le tiroir (60) rejoint la position de dégagement.

10. Ensemble (1) de raccordement de deux tubes (100, 200) comprenant deux dispositifs (10A, 10B) de raccordement selon l'une quelconque des revendications précédentes.

11. Ensemble (1) selon la revendication 10, dans lequel les deux dispositifs (10A, 10B) de raccordement sont identiques.
